# EUROPÄISCHE PATENTANMELDUNG

(11) **EP 4 512 386 A2**
(43) Veröffentlichungstag der Anmeldung: **26.02.2025**
(21) Anmeldenummer: 25151757.9
(22) Anmeldetag: 14.02.2022
(51) Int. Cl.: A61K 8/02

(54) **GLITTER UND DESSEN VERWENDUNG**

(30) Priorität: 17.02.2021 EP 21157546
(62) Teilanmeldung aus: 22156644.1
(71) Anmelder: Sigmund Lindner GmbH, 95485 Warmensteinach (DE)
(72) Erfinder: PSCHIERER, Erwin, 95506 Kastl (DE)
(74) Vertreter: Isarpatent

(57) **Zusammenfassung**

Die vorliegende Erfindung betrifft einen Glitter, umfassend eine Folie, die mit Metallpigmenten, insbesondere Aluminium-Pigmenten, beschichtet ist, ein Verfahren zu dessen Herstellung, dessen Verwendung in kosmetischen Produkten, Beschichtungswerkstoffen wie z. B. Farben, Lacken und Druckfarben, Klebstoffen und Knet- und/oder Formmassen, sowie kosmetische Produkte, Beschichtungswerkstoffe und Klebstoffe, und Knet- und/oder Formmassen, welche den Glitter umfassen.

## Beschreibung

Die vorliegende Erfindung betrifft einen Glitter, umfassend eine Folie, die mit Metallpigmenten, insbesondere Aluminium-Pigmenten, beschichtet ist, ein Verfahren zu dessen Herstellung, dessen Verwendung in kosmetischen Produkten, Beschichtungswerkstoffen wie z. B. Farben, Lacken und Druckfarben, Klebstoffen und Knet- und/oder Formmassen, sowie kosmetische Produkte, Beschichtungswerkstoffe und Klebstoffe, und Knet- und/oder Formmassen, welche den Glitter umfassen.

### Stand der Technik

Glitter finden vielfach Anwendung zur Erzeugung eines glitzernden Oberflächeneffektes und werden unter anderem und insbesondere in kosmetischen Artikeln, Farben, Lacken, Druckfarben, Klebstoffen oder Spielzeugen, wie z. B. Knet- und Formassen oder Fingerfarben, eingesetzt. So sind beispielsweise Formmassen umfassend Glitter aus EP 0 803 549 A2 oder EP 1 566 419 A1 bekannt.

Zur Herstellung derartiger Glitter werden üblicherweise Folien oder Filme aus Kunststoff verwendet, die mittels eines Schneidvorganges in einzelne, vergleichbar kleine Partikel geschnitten werden. Hauptsächlich werden derzeit Filme aus Polyethylenterephthalat für die Herstellung von Glitter verwendet. In geringerem Umfang werden Filme aus biologisch abbaubaren Filmen wie z. B. Polylactose, Celluloseacetat oder Cellulose für die Herstellung von Glitter verwendet. Ein beispielhaftes Verfahren zur Herstellung solcher Glitter ist in der DE 102010001971 A1 offenbart. Hierin werden Glitter offenbart, die von allen Seiten beschichtet werden. Ein weiteres Verfahren zum Beschichten von Glitter ist der EP 2 918 630 A1 zu entnehmen.

Meist sind Folien, die für die Herstellung von Glitter verwendet werden, mit Metallen (typischerweise Aluminium) beschichtet, wodurch eine reflektierende Oberfläche entsteht und ein ausgeprägter Glitzereffekt erzeugt werden kann. Typischerweise wird das Metall in einem Vakuumverfahren auf den Folien abgeschieden. Dieses Verfahren ist bekannt und wird kommerziell für verschiedene Folien angewendet. In EP 0769371 A2 wird die Herstellung von metallisierter Polyolefinfolie offenbart. In DE 69305336 T2 werden metallisierte Polyesterfolien genannt. DE 2856510 A1 offenbart ein Verfahren zur Herstellung einer Spiegelglanzmetallisierung auf einem Substrat.

Aus ökologischen Gesichtspunkten ist es wünschenswert, Glitter aus biologisch abbaubaren Materialien herzustellen. Bekannte biologisch abbaubare Folienmaterialien, wie z. B. Polylactose, Celluloseacetat und Cellulose sind kommerziell erhältlich mit Aluminiumschichten, die durch Vakuumverfahren auf die Oberfläche abgeschieden wurden.

Es ist bekannt, dass Cellulose und Cellulosederivate (z. B. Celluloseacetat) hohe Abbaugeschwindigkeiten unter verschiedenen Bedingungen (z. B. Kompostierung, Frischwasser oder Meerwasser) aufweisen. Deshalb bieten Folien auf Basis von Cellulose oder Cellulosederivaten sehr gute Voraussetzungen für die Herstellung von biologisch abbaubaren Glittern.

Es sind metallisierte Folien bekannt, die teilweise aus Cellulose bestehen und auf deren Oberfläche sich eine Aluminiumschicht befindet, die mittels eines Vakuumverfahrens aufgebracht wurde. Zum Beispiel werden diese Folien unter dem Markennamen Natureflex NM und Natureflex NKM (Fa. Futamura Chemical Co, Ltd.) vermarktet. Unter der Bezeichnung SiLiglam PURE BIO SPARKLE (Sigmund Lindner GmbH) werden Glitter vermarktet, die u. a. regenerierte Cellulose enthalten, die mit Aluminium mittels Vakuumverfahren beschichtet ist. EP 3552666 A1 lehrt die Herstellung von Glitter auf Basis von Celluloseacetat-Folien. In EP 3666830 A1 wird die Herstellung von Glitter auf Basis von vakuummetallisierter Zellulose-Folie offenbart.

Viele kosmetische Formulierungen sowie viele Farben, Lacke, weitere Beschichtungswerkstoffe, Klebstoffe oder Knet- und Formmassen enthalten Wasser. Aus ökologischen Gesichtspunkten ist es wünschenswert, in solchen wasserbasierten Formulierungen biologisch abbaubare Glitter zu verwenden. Aus der Praxis ist bekannt, dass Glitter, die aus vakuum-metallisierter Folie auf Basis von Cellulose hergestellt sind, in Kontakt mit Wasser eine geringe Formstabilität aufweisen und eine wellige Oberfläche ausbilden oder sich verformen. Außerdem ist die Lagerfähigkeit in wässrigen Formulierungen begrenzt, da Aluminium im wässrigen Milieu gemäß folgender Reaktionsgleichung in Lösung gehen kann.

2 Al + 6 H₂O → 2 Al³⁺(aq) + 6 OH⁻(aq) + 3 H₂

Ähnliche Reaktionen ergeben sich auch für andere Metalle, die in beschichteten Glittern Anwendung finden können.

Es besteht ein Bedarf an Glitter, welche biologisch abbaubar sind und in wasserbasierten Formulierungen formstabil bleiben. Darüber hinaus ist eine möglichst lange Lagerstabilität in wasserbasierten Formulierungen wünschenswert.

Der Erfindung liegt daher die Aufgabe zugrunde, biologisch abbaubare Glitter bereitzustellen, welche eine verbesserte Formstabilität und Lagerbeständigkeit in wasserbasierten Formulierungen aufweisen.

### Zusammenfassung der Erfindung

Es hat sich überraschenderweise gezeigt, dass Glitter, die mit Metallpigmenten, insbesondere Aluminium-Pigmenten, beschichtet sind, im Gegensatz zu vakuummetallisierten Glitter in wasserbasierten Formulierungen formstabil sind und eine deutlich längere Lagerstabilität aufweisen.

Bisher wurden für die Herstellung von biologisch abbaubarem Glitter hauptsächlich Folien mit einer Metallisierung verwendet, die mittels Vakuumbedampfung aufgebracht wurde. Es hat sich überraschend gezeigt, dass die Aufgabe der Erfindung, also biologisch abbaubare Glitter mit verbesserter Formbeständigkeit und Lagerbeständigkeit in wasserbasierten Formulierungen, gelöst werden kann, indem bei der Herstellung von Glitter Folien auf Basis biologisch abbaubarer Polymere verwendet werden, wobei die Folie mit Metallpigmenten, insbesondere Aluminium-Pigmenten, beschichtet ist und nicht mittels Vakuummetallisierung beschichtet ist.

Die vorliegende Erfindung betrifft somit einen beschichteten Glitter umfassend eine Folie, die eine Beschichtung umfassend Metall-Pigmente, insbesondere Aluminium-Pigmente, aufweist, wobei die Folie Cellulose und/oder ein Derivat davon umfasst.

Weiterhin betrifft die Erfindung die Verwendung des beschichteten Glitters in kosmetischen Formulierungen, Beschichtungswerkstoffen, Klebstoffen, und Knet- und/oder Formmassen, sowie kosmetische Formulierungen, Beschichtungswerkstoffe, Klebestoffe, und Knet- und/oder Formmassen umfassend den beschichteten Glitter.

Zudem offenbart ist ein Verfahren zur Herstellung eines beschichteten Glitters umfassend: Bereitstellen einer Folie, wobei die Folie Cellulose und/oder ein Derivat davon umfasst; Herstellen einer Beschichtung umfassend Metall-Pigmente, insbesondere Aluminium-Pigmente, auf mindestens einer Seite der Folie; und Herstellung des beschichteten Glitters; oder
Bereitstellen einer Folie, wobei die Folie Cellulose und/oder ein Derivat davon umfasst; Schneiden der Folie zu unbeschichteten Glitter-Partikeln geeigneter Größe; und
Beschichtung der unbeschichteten Glitter-Partikel mit Metall-Pigmenten, insbesondere Aluminium-Pigmenten.

Weitere Aspekte der vorliegenden Erfindung sind den abhängigen Ansprüchen und der detaillierten Beschreibung zu entnehmen.

### Beschreibung der Figuren

Die beiliegenden Zeichnungen sollen Ausführungsformen der vorliegenden Erfindung veranschaulichen und ein weiteres Verständnis dieser vermitteln. Im Zusammenhang mit der Beschreibung dienen sie der Erklärung von Konzepten und Prinzipien der Erfindung. Andere Ausführungsformen und viele der genannten Vorteile ergeben sich im Hinblick auf die Zeichnungen. Die Elemente der Zeichnungen sind nicht notwendigerweise maßstabsgetreu zueinander dargestellt. Gleiche, funktionsgleiche und gleich wirkende Elemente, Merkmale und Komponenten sind in den Figuren der Zeichnungen, sofern nichts anderes ausgeführt ist, jeweils mit denselben Bezugszeichen versehen.

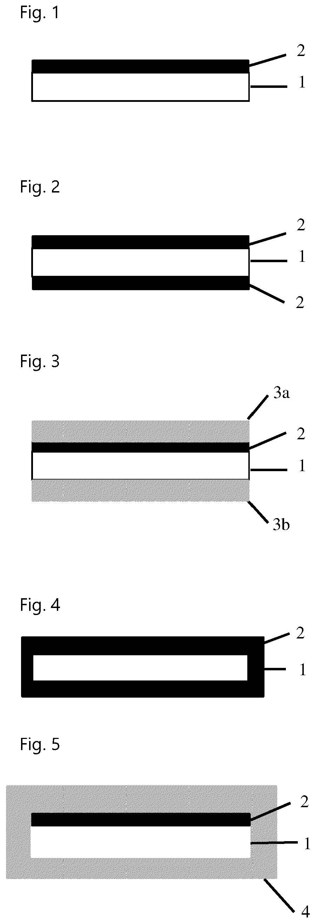

Figuren 1 bis 5 zeigen schematisch beispielhafte erfindungsgemäße Glitter.

### Detaillierte Beschreibung der Erfindung

### Definitionen

So nicht anderweitig definiert haben hierin verwendete technische und wissenschaftliche Ausdrücke dieselbe Bedeutung, wie sie von einem Fachmann auf dem Fachgebiet der Erfindung gemeinhin verstanden werden.

Mengenangaben im Rahmen der vorliegenden Erfindung beziehen sich auf Gew.%, soweit nicht anderweitig angegeben oder aus dem Kontext ersichtlich ist.

Glitter sind kleine Partikel mit verschiedensten Formen. Insbesondere weisen sie eine Größe, beispielsweise einen maximalen Durchmesser in einer Hauptausdehnungsrichtung des Partikels, von 0,02 mm bis 7,0 mm, bevorzugt 0,050 mm bis 6,0 mm, beispielsweise 0,06 mm bis 2,0 mm, z.B. 0,1 mm bis- 0,5 mm auf, beispielsweise von 100 µm bis 400 µm, auf. Hinsichtlich der Form sind die Glitterpartikel nicht besonders beschränkt und können beispielsweise als Plättchen, Nadeln, Quader, etc. vorliegen, oder zu bestimmten Formen gestanzt oder geschnitten sein, beispielsweise Sechsecken, Quadraten, Kreisen, Ovalen, Sternen, etc. Gemäß bestimmten Ausführungsformen sind die Glitter flach ausgeführt, beispielsweise als Plättchen mit verschiedensten Formen, z.B. auch sechseckig, rechteckig, quadratisch, sternförmig, rund, oval, etc., wobei bevorzugt die Dicke der Plättchen zwischen 4 µm und 50 µm, beispielsweise zwischen 5 µm und 45 µm, z.B. zwischen 10 µm und 35 µm, beispielshaft zwischen 14 µm und 23 µm liegen kann, und/oder die Größe, beispielsweise ein maximaler Durchmesser in einer Hauptausdehnungsrichtung der Glitter, von 0,02 mm bis 7,0 mm, bevorzugt 0,050 mm bis 6,0 mm, beispielsweise 0,06 mm bis 2,0 mm, z.B. 0,1 mm bis- 0,5 mm betragen kann. Gemäß bestimmten Ausführungsformen sind Glitter als Quadrate oder Rechtecke mit einer maximalen Länge von 0,02 mm bis 7,0 mm, bevorzugt 0,050 mm bis 6,0 mm, und/oder einer Dicke von 10,0 bis 50,0 µm vorgesehen.

Die vorliegende Erfindung betrifft in einem ersten Aspekt einen beschichteten Glitter, umfassend eine Folie, die eine Beschichtung umfassend Metall-Pigmente, insbesondere Aluminium-Pigmente, aufweist, wobei die Folie Cellulose und/oder ein Derivat davon umfasst.

Die Folie umfasst Cellulose und/oder ein Derivat davon. Diese sind biologisch abbaubare Polymere, welche eine verbesserte Formstabilität und Lagerbeständigkeit in wasserbasierten Formulierungen in Verbindung mit der Beschichtung umfassend Metall-Pigmente aufweisen. Die Cellulose und Derivate davon sind erfindungsgemäß nicht besonders beschränkt. Derivate von Cellulose sind beispielsweise Ester der Cellulose, wie Celluloseacetat, Cellulosepropionat, Celluloseacetatpropionat, Cellulosebutyrat, und/oder Celluloseacetatbutyrat. Daneben ist auch regenerierte Cellulose im Rahmen der Erfindung als Cellulosederivat anzusehen. Die Folie kann entsprechend beispielsweise Cellulose, regenerierte Cellulose bzw. Hydratcellulose (z.B. Zellglas, Cellophan, Cellulosehydrat), Celluloseacetat, Pergamin, Zellstoff, oder ein Gemisch aus zwei oder mehreren davon, als Cellulose und Derivat davon umfassen. Gemäß bestimmten Ausführungsformen umfasst die Folie Cellulose. Gemäß bestimmten Ausführungsformen umfasst die Folie Celluloseacetat.

Es ist hierbei jedoch nicht ausgeschlossen, dass die Folie weitere Bestandteile umfasst, beispielsweise mindestens ein weiteres Polymer wie ein Cellulose-Derivat neben Cellulose, wie oben angegeben, aber auch beispielsweise Polymilchsäure (PLA), Polyvinylalkohol, Polyhydroxyalkanoat (PHA), und/oder Polybutylenadipat-Terephthalat (BTA-Copolyester).

Gemäß bestimmten Ausführungsformen umfasst die Folie wenigstens 60 Gew.% Cellulose und/oder ein Derivat davon, bezogen auf das Gewicht der Folie, bevorzugt wenigstens 70 Gew.% Cellulose und/oder ein Derivat davon, bezogen auf das Gewicht der Folie, weiter bevorzugt wenigstens 80 Gew.% Cellulose und/oder ein Derivat davon, bezogen auf das Gewicht der Folie, noch weiter bevorzugt 90 Gew.% Cellulose und/oder ein Derivat davon, bezogen auf das Gewicht der Folie. Gemäß bestimmten Ausführungsformen besteht die Folie im Wesentlichen aus Cellulose und/oder einem Derivat davon, also umfasst wenigstens 95 Gew.%, 98 Gew.%, 99 Gew.%, 99,5 Gew.% oder sogar 99,9 Gew.% Cellulose und/oder ein Derivat davon, bezogen auf das Gewicht der Folie, oder besteht sogar aus Cellulose und/oder einem Derivat davon.

Gemäß bestimmten Ausführungsformen umfasst die Folie wenigstens 60 Gew.% Cellulose, bezogen auf das Gewicht der Folie, bevorzugt wenigstens 70 Gew.% Cellulose, bezogen auf das Gewicht der Folie, weiter bevorzugt wenigstens 80 Gew.% Cellulose, bezogen auf das Gewicht der Folie, noch weiter bevorzugt 90 Gew.% Cellulose, bezogen auf das Gewicht der Folie. Gemäß bestimmten Ausführungsformen besteht die Folie im Wesentlichen aus Cellulose, also umfasst wenigstens 95 Gew.%, 98 Gew.%, 99 Gew.%, 99,5 Gew.% oder sogar 99,9 Gew.% Cellulose, bezogen auf das Gewicht der Folie, oder besteht sogar aus Cellulose.

Gemäß bestimmten Ausführungsformen umfasst die Folie wenigstens 60 Gew.% Celluloseacetat, bezogen auf das Gewicht der Folie, bevorzugt wenigstens 70 Gew.% Celluloseacetat, bezogen auf das Gewicht der Folie, weiter bevorzugt wenigstens 80 Gew.% Celluloseacetat, bezogen auf das Gewicht der Folie, noch weiter bevorzugt 90 Gew.% Celluloseacetat, bezogen auf das Gewicht der Folie. Gemäß bestimmten Ausführungsformen besteht die Folie im Wesentlichen aus Celluloseacetat, also umfasst wenigstens 95 Gew.%, 98 Gew.%, 99 Gew.%, 99,5 Gew.% oder sogar 99,9 Gew.% Celluloseacetat, bezogen auf das Gewicht der Folie, oder besteht sogar aus Cellu loseacetat.

Daneben können jedoch auch weitere Additive in der Folie vorhanden sein, welche nicht besonders beschränkt sind. Auch kann die Folie keine Additive enthalten. Alternativ oder zusätzlich kann die Folie gemäß bestimmten Ausführungsformen Additive enthalten, welche z.B. als Antiblock- und/oder Gleithilfsmittel wirken können. Diese Antiblock und/oder Gleithilfsmittel sind nicht besonders beschränkt und können beispielsweise amorphe Kieselsäuren, kristalline Kieselsäuren, Fettsäureamide und/oder Talk umfassen. Die Zugabemenge für solche Antiblock- und/oder Gleithilfsmittel kann gemäß bestimmten Ausführungsformen 0 - 2,5 Gew.%, bevorzugt 0 - 1,5 Gew.%, weiter bevorzugt 0 - 1 Gew.% betragen, bezogen auf das Gewicht der Folie.

Alternativ oder zusätzlich kann die Folie gemäß bestimmten Ausführungsformen Pigmente und/oder Füllstoffe enthalten, welche z.B. die Farbe oder die mechanischen Eigenschaften beeinflussen können. Diese Pigmente und/oder Füllstoffe sind nicht besonders beschränkt und können beispielsweise Calciumcarbonat, Bariumsulfat, anorganische Farbpigmente, organische Farbpigmente und/oder Mica umfassen. Der Massenanteil ist nicht besonders beschränkt und kann hierbei bevorzugt weniger als 40 Gew.%, weiter bevorzugt weniger als 30 Gew.%, noch weiter bevorzugt weniger als 20 Gew.%, und insbesondere bevorzugt weniger als 10 Gew.%, beispielsweise weniger als 5 Gew.%, oder sogar weniger als 1 Gew.%, betragen, bezogen auf das Gewicht der Folie. Auch kann die Folie keine Pigmente und/oder Füllstoffe enthalten. Alternativ oder zusätzlich kann die Folie gemäß bestimmten Ausführungsformen Weichmacher und/oder Feuchtemittel enthalten. Diese Weichmacher und/oder Feuchtemittel sind nicht besonders beschränkt und können beispielsweise Glycerin, Harnstoff und/oder Triacetin umfassen. Die Zugabemenge für solche Weichmacher und/oder Feuchtemittel ist nicht besonders beschränkt und kann gemäß bestimmten Ausführungsformen 0 - 30 Gew.%, bevorzugt 0 - 15 Gew.%, weiter bevorzugt 0 - 10 Gew.% betragen, bezogen auf das Gewicht der Folie.

Die Folie weist eine Beschichtung auf, welche Metall-Pigmente, insbesondere Aluminium-Pigmente, umfasst. Obgleich Aluminium-Pigmente in der Beschichtung bevorzugt sind, können auch alternativ oder zusätzlich andere Pigmente vorhanden sein. Als Metall-Pigmente eignen sich beispielsweise Aluminium-Pigmente, Kupfer-Pigmente, Silber-Pigmente, und/oder Gold-Pigmente, und/oder Legierungen der genannten Metalle.

Das Aufbringen der Beschichtung ist hierbei nicht besonders beschränkt, und die Beschichtung kann auf einer Seite, auf zwei gegenüberliegenden Seiten, auf der gesamten Folie oder auch auf andere Art und Weise, beispielsweise auch teilweise, vorhanden sein. Gemäß bestimmten Ausführungsformen ist die Beschichtung zumindest auf einer Seite der Folie, beispielsweise einer Oberseite als Fläche, vorhanden. Die Aufbringung kann auf beliebige Weise erfolgen, beispielsweise aus der Gas- und/oder flüssigen Phase - wobei die flüssige Phase auch Feststoffe umfassen darf, beispielsweise aus Suspension, etc. Gemäß bestimmten Ausführungsformen erfolgt die Beschichtung aus flüssiger Phase, beispielsweise unter Verwendung einer Suspension, einer Dispersion, etc.

Gemäß bestimmten Ausführungsformen werden erfindungsgemäß die Metall-Pigmente mit einem Beschichtungsmaterial aufgebracht. Entsprechend kann eine Beschichtung im erfindungsgemäßen beschichteten Glitter auch mindestens ein Beschichtungsmaterial umfassen, welches nicht besonders beschränkt ist.

Das Beschichtungsmaterial, das die Metall-Pigmente, insbesondere Aluminium-Pigmente, enthält, ist nicht besonders beschränkt und kann übliche Beschichtungsmaterialien für Materialien auf Cellulose-Basis und/oder für Glitter umfassen. Gemäß bestimmten Ausführungsformen kann das Beschichtungsmaterial ein Material auf Basis von Cellulose oder modifizierter Cellulose, bevorzugt auf Cellulosenitrat-Basis, Celluloseacetatbutyrat-Basis, Celluloseacetatpropionat-Basis und/oder Celluloseacetat-Basis, und/oder auf Polyvinylalkohol-Basis und/oder Schellackbasis-Basis und/oder Polyurethan-Basis und/oder Basis von Polyvinylpyrrolidon, und/oder auf Basis von Naturharzen, bevorzugt Dammar und/oder Kopal und/oder Gum Sandarak und/oder Gum Arabicum und/oder auf Lignin-Basis und/oder auf Zein-Basis, umfassen oder daraus hergestellt sein. Die genannten Bindemittel können gemäß bestimmten Ausführungsformen einen Anteil von 10 - 99,9 Gew.% aufweisen, bezogen auf das Gewicht des Beschichtungsmaterials. Zusätzlich kann das Beschichtungsmaterial gemäß bestimmten Ausführungsformen Pigmente und/oder Füllstoffe enthalten, welche z.B. die Farbe oder die technischen Eigenschaften beeinflussen können. Diese Pigmente und/oder Füllstoffe Additive sind nicht besonders beschränkt und können beispielsweise Calciumcarbonat, Bariumsulfat, anorganische Farbpigmente, organische Farbpigmente und/oder Mica umfassen. Der Massenanteil kann hierbei bevorzugt weniger als 40 Gew.%, weiter bevorzugt weniger als 30 Gew.%, noch weiter bevorzugt weniger als 20 Gew.%, und insbesondere bevorzugt weniger als 10 Gew.%, beispielsweise weniger als 5 Gew.%, oder sogar weniger als 1 Gew.%, betragen, bezogen auf das Gewicht des Beschichtungsmaterials. Auch kann die Beschichtung keine Pigmente und/oder Füllstoffe enthalten.

Alternativ oder zusätzlich kann das Beschichtungsmaterial gemäß bestimmten Ausführungsformen Additive enthalten, welche die optischen und/oder technischen Eigenschaften beeinflussen. Zum Beispiel können die Additive als Gleithilfsmittel und/oder Entschäumer wirken. Diese Additive sind nicht besonders beschränkt und können beispielsweise Wachse und/oder grenzflächenaktive Substanzen (z. B. Silikone, Tenside) sein. Die Zugabemenge für solche Additive kann gemäß bestimmten Ausführungsformen 0 - 10 Gew.%, bevorzugt 0 - 5 Gew.%, weiter bevorzugt 0 - 2 Gew.% betragen, bezogen auf das Gewicht des Beschichtungsmaterials. Auch kann die Beschichtung keine weiteren Additive enthalten. Alternativ oder zusätzlich kann das Beschichtungsmaterial gemäß bestimmten Ausführungsformen Weichmacher und/oder Feuchtemittel enthalten. Diese Weichmacher und/oder Feuchtemittel sind nicht besonders beschränkt und können beispielsweise Glycerin, Harnstoff und/oder Triacetin umfassen. Die Zugabemenge für solche Weichmacher und/oder Feuchtemittel ist nicht besonders beschränkt und kann gemäß bestimmten Ausführungsformen 0 - 30 Gew.%, bevorzugt 0 - 15 Gew.%, weiter bevorzugt 0 - 10 Gew.% betragen, bezogen auf das Gewicht des Beschichtungsmaterials.

Gemäß bestimmten Ausführungsformen weist die Beschichtung eine Trockenschichtdicke von 0,1 µm bis 10 µm, bevorzugt von 0,5 bis 5 µm, weiter bevorzugt von 1 µm bis 3 µm auf. Diese wird gemäß bestimmten Ausführungsformen auf nur einer Seite, mehreren Seiten oder jeweils einer Seite der Folie nach Trocknung, beispielsweise im Gasstrom oder im Trockenschrank, erzielt. Die Trockenschichtdicke kann hierbei beispielsweise mittels spektraler Reflexion, beispielsweise mit einem Messgerät der Firma Filmetrics, bestimmt werden. Obgleich praktisch nicht beliebig dünne Schichten aufgetragen werden können, kann mit dünneren Schichten ein verbesserter Glanzeffekt erzielt werden.

Der Gehalt an Metall-Pigmenten, insbesondere Aluminium-Pigmenten, kann gemäß bestimmten Ausführungsformen 0,01 - 10 Gew.%, bevorzugt 0,01 - 5 Gew.%, weiter bevorzugt 0,01 - 1 Gew.%, und insbesondere bevorzugt 0,01 - 0,5 Gew.% betragen, bezogen auf das Gewicht der Folie. Hierdurch wird die Beständigkeit der Glitter gegen Wasser besonders erhöht. Gemäß bestimmten Ausführungsformen sind die Glitter mindestens zweimal so groß, bezogen auf eine zu beschichtende Oberfläche auf einer Seite der Glitter, wie die Metall-Pigmente.

Darüber hinaus kann der erfindungsgemäße beschichtete Glitter auch ein oder mehrere weitere, z.B. farb- und/oder effektgebende oder auch anders geartete - z.B. zur Verbesserung der UV-Beständigkeit, Abriebsfestigkeit und/oder Wasserbeständigkeit, Schichten umfassen, wie sie dem Fachmann bekannt sind und die ein Fachmann geeignet beschichten kann, beispielsweise aus der Gasphase und/oder aus Flüssigkeit/Lösung. Diese Beschichtungen sind nicht besonders beschränkt und können beispielsweise auf Basis von Cellulose oder modifizierter Cellulose, bevorzugt auf Cellulosenitrat-Basis, Celluloseacetatbutyrat-Basis, Celluloseacetatpropionat-Basis und/oder Celluloseacetat-Basis, und/oder auf Polyvinylalkohol-Basis und/oder Schellack-Basis und/oder Polyurethan-Basis und/oder Polyvinylpyrrolidon-Basis, und/oder auf Basis von Naturharzen, bevorzugt Dammar und/oder Kopal und/oder Gum Sandarak und/oder Gum Arabicum und/oder Zein, hergestellt sein. Die genannten Bindemittel können gemäß bestimmten Ausführungsformen einen Anteil von 10 - 99,9 Gew.% aufweisen, bezogen auf das Gewicht des Beschichtungsmaterials.

Gemäß bestimmten Ausführungsformen weist die Folie der erfindungsgemäße Glitter eine Dicke von 5 µm oder mehr, bevorzugt von mehr als 10 µm, weiter bevorzugt von mehr als 13 µm, und/oder eine Dicke von 50 µm oder weniger, beispielsweise weniger als 40 µm, bevorzugt 36 µm oder weniger, auf.

Die Folie kann im erfindungsgemäßen beschichteten Glitter gefärbt oder ungefärbt sein, ist gemäß bestimmten Ausführungsformen jedoch ungefärbt. Zudem kann in bestimmten Ausführungsformen auf die Beschichtung umfassend Metall-Pigmente, insbesondere Aluminium-Pigmente, oder die Folie eine farb- und/oder effektgebende oder auch anders geartete Schicht aufgebracht werden. Bevorzugt ist die Beschichtung umfassend Metall-Pigmente, insbesondere Aluminium-Pigmente, direkt auf die Folie aufgebracht, insbesondere auf allen Seiten der Folie, wobei jedoch auch eine Beschichtung auf einer Seite bereits die Beständigkeit erhöht. So die Beschichtung umfassend Metall-Pigmente, insbesondere Aluminium-Pigmente, nicht auf allen Seiten der Folie aufgebracht ist, ist auch eine Beschichtung - zumindest teilweise, der Folie mit einer farb- und/oder effektgebenden oder auch anders gearteten Schicht möglich.

Gemäß bestimmten Ausführungsformen kann die Folie in einem erfindungsgemäßen Glitter auch mit einer Hologramm-Prägung hergestellt werden, wie sie bei herkömmlichem Glitter bekannt ist, beispielsweise aus der US 5,810,957 oder EP 2163381 A, wobei auf die beiden Dokumente hinsichtlich der Holgramm-Prägung Bezug genommen wird. Überraschenderweise lässt sich die Holgramm-Prägung auch auf mit Metall-Pigmenten, insbesondere Aluminium-Pigmenten, beschichteten Folien realisieren. Zur Herstellung können hierbei gängige Verfahren verwendet werden, wie das sogenannte Soft-Embossing und das Hard-Embossing.

Nachfolgend werden beispielhafte erfindungsgemäße beschichtete Glitter näher erläutert anhand beispielhafter Ausführungsformen, welche in Figuren 1 bis 5 gezeigt sind. Die Figuren zeigen hierbei schematisch Schnittansichten durch Glitter mit einem erfindungsgemäßen Aufbau.

Gemäß einer ersten beispielhaften Ausführungsform, die in Fig. 1 dargestellt ist, können beispielsweise aus einer Folie umfassend Cellulose und/oder ein Derivat davon, beispielsweise umfassend Cellulose und/oder Celluloseacetat, bestehende Partikel 1 einseitig mit einer Beschichtung umfassend Metall-Pigmente, beispielsweise mit einer Celluloseacetat-Schicht 2, die Aluminium-Pigmente beinhaltet, beschichtet sein. Die Beschichtung umfassend Metall-Pigmente, beispielsweise aus Celluloseacetat und Aluminium-Pigmenten, wird z.B. aus flüssiger Phase auf eine Folie umfassend Cellulose und/oder ein Derivat davon, die beispielsweise auch transparent sein kann, aufgebracht, beispielsweise mittels einem Tiefdruckverfahren. Aus der beschichteten Folie können durch Schneiden dann die Glitterpartikel hergestellt werden.

Gemäß einer weiteren Ausführungsform, die in Fig. 2 dargestellt ist, können beispielsweise aus einer Folie umfassend Cellulose und/oder ein Derivat davon, beispielsweise umfassend Cellulose und/oder Celluloseacetat, bestehende Partikel 1 auf zwei gegenüberliegenden Seiten, beispielsweise mit einer Celluloseacetat-Schicht 2, die Aluminium-Pigmente beinhaltet, beschichtet sein. Die Beschichtung aus Celluloseacetat und Aluminium-Pigmenten kann aus flüssiger Phase auf eine Folie umfassend Cellulose und/oder ein Derivat davon, beispielsweise umfassend Cellulose und/oder Celluloseacetat, aufgebracht werden, beispielsweise mittels Tiefdruckverfahren, wobei dann beide Seiten der Folie bedruckt werden können. Aus der beschichteten Folie können durch Schneiden die Glitterpartikel hergestellt werden.

Darüber hinaus können die aus einer Folie umfassend Cellulose und/oder ein Derivat davon, beispielsweise umfassend Cellulose und/oder Celluloseacetat, bestehenden Partikel 1, die einseitig mit einer Aluminium-Pigmente beinhaltenden Beschichtung, z.B. einer Celluloseacetat-Schicht 2, beschichtet sind, in einer weiteren Ausführungsform, wie in Fig. 3 gezeigt, zusätzlich auf zwei gegenüberliegenden Seiten gleichmäßig mit weiteren Schichten 3a, 3b aus beispielsweise Celluloseacetat beschichtet sein. Diese können beispielsweise wie die Celluloseacetat-Schicht 2, die Aluminium-Pigmente umfasst, aufgedruckt werden.

Alternativ können aus einer Folie umfassend Cellulose und/oder ein Derivat davon, beispielsweise umfassend Cellulose und/oder Celluloseacetat, bestehende Partikel 1 in einer weiteren Ausführungsform, wie in Fig. 4 gezeigt, auf allen Seiten gleichmäßig mit einer Beschichtung umfassend Metall-Pigmente, beispielsweise mit einer Celluloseacetat-Schicht 2, die Aluminium-Pigmente beinhaltet, beschichtet sein. Dies kann beispielsweise durch Beschichtung in flüssiger oder Gasphase erfolgen.

Darüber hinaus können die aus einer Folie umfassend Cellulose und/oder ein Derivat davon, beispielsweise umfassend Cellulose und/oder Celluloseacetat, bestehenden Partikel 1, die einseitig mit einer Beschichtung umfassend Metall-Pigmente, beispielsweise mit einer Aluminium-Pigmente beinhaltenden Celluloseacetat-Schicht 2, beschichtet sind, in einer weiteren Ausführungsform, wie in Fig. 5 gezeigt, zusätzlich auf allen Seiten gleichmäßig mit einer weiteren Schicht 4 aus beispielsweise Celluloseacetat beschichtet sein. Die weitere Schicht kann hierbei beispielsweise in einer Wirbelschicht oder auf andere Weise aufgebracht werden, beispielsweise auch nach dem Schneiden der mit Schicht 2 beschichteten Partikel.

In den Figuren 1 bis 5 kann die Folie gemäß alternativen Ausgestaltungen auch gefärbt sein. In den Figuren 3 bis 5 können zudem die Schichten 3a, 3b und/oder 4 gemäß alternativen Ausgestaltungen auch gefärbt sein.

Ein weiterer Aspekt der vorliegenden Erfindung ist auf die Verwendung von erfindungsgemäßen beschichteten Glittern in einem kosmetischen Produkt gerichtet. Das kosmetische Produkt ist hierbei nicht besonders beschränkt. Kosmetische Produkte umfassen hierbei beispielsweise Pasten, Salben, Cremes, Emulsionen, Lösungen, Lippenstift, Lip Gloss, Mascara, Mousse, Eye Shadow, Eye Liner, Puder, gepresste Puder, loses Glitterpulver, Nagellack, Seifen, Shampoo, Sonnenschutzmittel, Lotionen, Aerosol-Sprays, usw., welche die Glitter in üblichen Mengen in den Formulierungen enthalten können.

Zudem offenbart ist ein kosmetisches Produkt, welches erfindungsgemäße beschichtete Glitter umfasst. Das kosmetische Produkt ist hierbei nicht besonders beschränkt, und es kann sich beispielsweise um eine Paste, eine Salbe, eine Creme, eine Emulsion, eine Lösung, einen Lippenstift, Lip Gloss, Mascara, Mousse, Eye Shadow, Eye Liner, Puder, gepressten Puder, loses Glitterpulver, Nagellack, Seife, Shampoo, Sonnenschutzmittel, eine Lotion, ein Aerosol-Spray, usw., handeln, wobei die Glitter in üblichen Mengen im kosmetischen Produkt enthalten sein können, beispielsweise zwischen 0,01 und 75 Gew.%, z.B. zwischen 1 und 10 Gew.% bezogen auf das kosmetische Produkt, oder sogar bis zu 100 Gew.% im Falle von Puder und losem Glitterpulver. Daneben können in den kosmetischen Produkten die üblichen Bestandteile vorhanden sein, wie Trägerstoffe, Füllstoffe, Öle, Wachse, Fette, Emulgatoren, Antioxidationen, Filmbildner, Geruchs- und/oder Geschmacksstoffe, Stabilisatoren, Lösemittel, Tenside, Konservierungsmittel, Verdicker, rheologische Additive, Farbstoffe, Vitamine, Puffersubstanzen, kosmetische Wirkstoffe, hautaktive Stoffe, z.B. hautpflegende Stoffe, UV-Filter, etc., welche alle nicht besonders beschränkt sind. Die kosmetischen Produkte können beispielsweise hydrophiler, hydrophober und/oder lipophiler Natur sein. Entsprechende Bestandteile sind beispielsweise aus der DE 102005055576 A1 bekannt, auf die beispielhaft in Bezug auf kosmetische Formulierungen zur Herstellung kosmetischer Produkte Bezug genommen wird.

Die erfindungsgemäßen Glitter sind mit einer Vielzahl an typischen Formulierungen aus den Bereichen Farben, Lacke, Klebstoffe, oder Form- und Knetmassen kompatibel. Offenbart ist entsprechend die Verwendung der erfindungsgemäßen beschichteten Glitter in Beschichtungswerkstoffen, beispielsweise auch Beschichtungssystemen, vorzugsweise aus dem Bereich der Lacke, Farben oder Druckfarben, wobei diese hier nicht besonders beschränkt ist und die Glitter in üblichen Mengen enthalten sein können.

Beispielsweise sind für die Verwendung in Druckfarben für z. B. den Tiefdruck oder Flexodruck eine Vielzahl von wasserverdünnbaren oder wasserbasierten Druckfarben und Überdrucklacke geeignet, wie sie z. B. von den Firmen Marabu, Pröll, Hartmann, Siegwerk, Rotoflex, GSB-Wahl und/oder Coates Screen vertrieben werden, und in denen erfindungsgemäße Glitter eingesetzt werden können. Die Trocknung und/oder Aushärtung der entsprechenden Beschichtungswerkstoffe kann beispielsweise thermisch erfolgen oder durch Strahlenhärtung.

Für die Verwendung der erfindungsgemäßen beschichteten Glitter in Lacken und Farben eignen sich eine Vielzahl an wasserverdünnbaren oder wasserbasierten Ansätzen, welche nicht besonders beschränkt sind. Die Applikation der Beschichtungsstoffe ist nicht besonders beschränkt und erfolgt typischerweise durch Streichen, Rollen, im Spritzverfahren, als Aerosol-Spray oder ein anderes geeignetes Verfahren. Ebenso geeignet sind Fingerfarben, Bastelfarben oder Künstlerfarben.

Weiterhin offenbart ist ein Beschichtungswerkstoff, der den erfindungsgemäßen beschichteten Glitter umfasst. Die Menge der Glitter im Beschichtungswerkstoff ist hierbei nicht besonders beschränkt und kann übliche Mengen umfassen, abhängig vom jeweiligen Beschichtungswerkstoff. In Beschichtungswerkstoffen werden die erfindungsgemäßen Glitter beispielsweise in Konzentrationen von 0,1 Gew.% bis 70 Gew.%, bevorzugt in Konzentrationen von 0,5 Gew.% bis 50 Gew.%, besonders bevorzugt in Konzentrationen von 0,5 Gew.% bis 10 Gew.%, eingesetzt, bezogen auf den Beschichtungswerkstoff. Als Beschichtungsstoffe kommen beispielsweise Farben oder Lacke einschließlich Druckfarben in Betracht, beispielsweise auch Fingerfarben, Bastelfarben oder Künstlerfarben.

Daneben offenbart ist die Verwendung der erfindungsgemäßen beschichteten Glitter in einem Klebstoff, welcher nicht besonders beschränkt ist. Für die Verwendung in beispielsweise wasserverdünnbaren oder wasserbasierten Klebstoffen, wie z. B. Bastelklebstoff, Universalklebstoff oder Papierklebstoff, sind die geeigneten Systeme hier nicht besonders beschränkt.

Weiterhin offenbart ist ein Klebstoff, der den erfindungsgemäßen beschichteten Glitter umfasst. Die Menge der Glitter im Klebstoff ist hierbei nicht besonders beschränkt und kann übliche Mengen umfassen, abhängig vom jeweiligen Klebstoff. In Klebstoffen werden die erfindungsgemäßen Glitter beispielsweise in Konzentrationen von 0,1 Gew.% bis 70 Gew.%, bevorzugt in Konzentrationen von 0,5 Gew.% bis 50 Gew.%, besonders bevorzugt in Konzentrationen von 0,5 Gew.% bis 10 Gew.%, eingesetzt, bezogen auf den Klebstoff. Als Klebstoffe kommen beispielsweise wasserverdünnbare oder wasserbasierte Klebstoffen, wie z. B. Bastelklebstoff, Universalklebstoff oder Papierklebstoff, in Betracht.

Ebenso geeignet sind die erfindungsgemäßen beschichteten Glitter für die Verwendung in Knetmassen und Formmassen, z.B. solchen, die Wasser enthalten, wie z. B. Spiel- und Schulknete oder Pappmaché, und welche nicht besonders beschränkt sind.

Zudem offenbart ist eine Knet- oder Formmasse, die den erfindungsgemäßen beschichteten Glitter umfasst. Die Knet- oder Formmasse ist nicht besonders beschränkt und umfasst beispielsweise Wasser enthaltende Knetmassen und Formmassen, wie z. B. Spiel- und Schulknete sowie Pappmaché. Die Menge der Glitter in der Knet- oder Formmasse ist hierbei nicht besonders beschränkt und kann übliche Mengen umfassen, abhängig von der jeweiligen Knet- oder Formmasse. In Knet- oder Formmassen werden die erfindungsgemäßen beschichteten Glitter beispielsweise in Konzentrationen von 0,1 Gew.% bis 60 Gew.%, bevorzugt in Konzentrationen von 0,5 Gew.% bis 20 Gew.%, besonders bevorzugt in Konzentrationen von 0,5 Gew.% bis 10 Gew.%, eingesetzt, bezogen auf die Knet- oder Formmasse.

Weiterhin offenbart ist ein Verfahren zur Herstellung eines beschichteten Glitters umfassend: Bereitstellen einer Folie, wobei die Folie Cellulose und/oder ein Derivat davon umfasst; Herstellen einer Beschichtung umfassend Metall-Pigmente, insbesondere Aluminium-Pigmente, auf mindestens einer Seite der Folie, beispielsweise auch zwei oder mehr Seiten der Folie oder auf der ganzen Folie; und Herstellung des beschichteten Glitters; oder
Bereitstellen einer Folie, wobei die Folie Cellulose und/oder ein Derivat davon umfasst; Schneiden der Folie zu unbeschichteten Glitter-Partikeln geeigneter Größe; und Beschichtung der unbeschichteten Glitter-Partikel mit Metall-Pigmenten, insbesondere Aluminium-Pigmenten, beispielsweise auf mindestens einer Seite der Folie, beispielsweise auch zwei oder mehr Seiten der Folie oder auf der ganzen Folie.

Mit dem erfindungsgemäßen Verfahren werden insbesondere erfindungsgemäße beschichtete Glitter hergestellt. Insofern können Aspekte, die in Zusammenhang mit dem erfindungsgemäßen beschichteten Glitter offenbart wurden, auch entsprechend beim erfindungsgemäßen Verfahren Anwendung finden, beispielsweise hinsichtlich Beschaffenheit der Folie, Beschichtung, etc., oder etwaiger weiterer Schichten, die ggf. geeignet weiter aufgebracht werden können, und vice versa. Auch können einzelne Ausführungsformen, welche bereits in Zusammenhang mit dem beschichteten Glitter beschrieben wurden, auch entsprechend im erfindungsgemäßen Verfahren Anwendung finden, beispielsweise hinsichtlich Gewichtsanteilen von bestimmten Stoffen, etc., und vice versa.

Das Verfahren zur Herstellung der erfindungsgemäßen Glitter ist darüber hinaus nicht besonders beschränkt. Eine Folie, umfassend eine Beschichtung mit Metall-Pigmenten, insbesondere Aluminium-Pigmenten, kann hierbei beispielsweise geeignet zu Partikeln geeigneter Größe geschnitten und ggf. auf übliche Weise mit Polymeren - wie beispielhaft oben beschrieben, Pigmenten, etc., beschichtet werden. Das Aufbringen verschiedener Beschichtungen ist nicht besonders beschränkt und kann beispielsweise auf die Folie (als Rollenware) und/oder auf die Partikel, beispielsweise in einem Wirbelbett, erfolgen.

Das Bereitstellen der Folie ist im erfindungsgemäßen Verfahren nicht besonders beschränkt.

Das Herstellen einer Beschichtung kann auf geeignete Weise erfolgen, beispielsweise durch Aufbringung der Metall-Pigmente, ggf. mit einem Beschichtungsmaterial und/oder ggf. weiteren Komponenten, wie oben in Zusammenhang mit dem erfindungsgemäßen Glitter beschrieben, auf mindestens einer Seite der Folie. Die Aufbringung kann auf beliebige Weise erfolgen, beispielsweise aus der Gas- und/oder flüssigen Phase - wobei die flüssige Phase auch Feststoffe umfassen darf, beispielsweise aus Suspension, etc. Gemäß bestimmten Ausführungsformen erfolgt die Beschichtung aus flüssiger Phase, beispielsweise unter Verwendung einer Suspension, einer Dispersion, etc. Die Beschichtung kann hierbei gemäß bestimmten Ausführungsformen auf einer Seite, auf zwei gegenüberliegenden Seiten, auf der gesamten Folie oder in anderer Weise erfolgen. Das Aufbringen der Metall-Pigmente, insbesondere Aluminium-Pigmente, auf die Folie erfolgt bevorzugt aus flüssiger Phase. Die Metall-Pigmente, insbesondere Aluminium-Pigmente, können beispielsweise als Dispersion in einem Lösemittel oder Lösemittelgemisch vorliegen. Alternativ können die Metallpigmente, insbesondere Aluminium-Pigmente, Bestandteil eines flüssigen Beschichtungsmaterials sein, das polymere Bindemittel, Additive, Füllstoffe oder Lösemittel enthalten kann. Das Aufbringen einer flüssigen Phase, die die Metall-Pigmente, insbesondere Aluminium-Pigmente, enthält, kann mittels dem Fachmann bekannte Beschichtungsverfahren, wie z. B. Tiefdruck, Flexodruck oder Siebdruck erfolgen, ist jedoch nicht auf ein bestimmtes Verfahren beschränkt.

Geeignete Metall-Pigmente, insbesondere Aluminium-Pigmente, sind nicht besonders beschränkt und werden beispielsweise von der Firma Eckart GmbH (Markennamen z. B. Rotovario, Stapa, Silveroto, Metalure, Standart, Visionaire) oder Firma Carl Schlenk AG (Markennamen z. B. Alushine, Alustar, Aquamet, Decomet, Alegrace) hergestellt und vertrieben.

Das Herstellen des beschichteten Glitters umfasst beispielsweise ein Schneiden der beschichteten Folie, welches nicht besonders beschränkt ist.

Im alternativen Verfahren ist nach dem Schneiden der Folie die Beschichtung der unbeschichteten Glitter-Partikel mit Metall-Pigmenten, insbesondere Aluminium-Pigmenten nicht besonders beschränkt, und kann beispielsweise aus flüssiger Phase, wie beispielsweise bei der Herstellung einer Beschichtung beschrieben, oder durch Eintauchen, etc., erfolgen, oder auch aus der Flüssig-Gasphase, beispielsweise durch Beschichtung mit einem Spray, Aerosol, etc., oder auf andere geeignete Weise.

Gemäß bestimmten Ausführungsformen erfolgt die Beschichtung aus flüssiger Phase, beispielsweise unter Verwendung einer Suspension, einer Dispersion, etc. Gemäß bestimmten Ausführungsformen erfolgt die Beschichtung mit Metall-Pigmenten, insbesondere Aluminium-Pigmenten, aus flüssiger Phase, insbesondere auf der Folie.

Die obigen Ausführungsformen, Ausgestaltungen und Weiterbildungen lassen sich, sofern sinnvoll, beliebig miteinander kombinieren. Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmalen der Erfindung. Insbesondere wird der Fachmann auch Einzelaspekte als Verbesserungen oder Ergänzungen zu der jeweiligen Grundform der vorliegenden Erfindung hinzufügen.

Die Erfindung wird im Anschluss mit Bezug auf verschiedene Beispiele davon weiter im Detail erläutert. Die Erfindung ist jedoch nicht auf diese Beispiele beschränkt.

### Beispiele

### Beispiel 1: Beschichtung im Tiefdruckverfahren

Erfindungsgemäße Glitter gemäß Figur 1 wurden durch Beschichten einer Folie, enthaltend 87 Gew.% Cellulose, 10 Gew.% Glycerin und 3 Gew.% Harnstoff, mit einer Dicke von 17 µm bis 23 µm mit Aluminium-Pigmenten durch Bedrucken im Tiefdruckverfahren auf einer Seite und anschließendes Schneiden auf eine Größe von 200 µm +/- 20 µm hergestellt. Für die Herstellung des Beschichtungsmaterials werden 100 g Celluloseacetat in 1000 g Methylethylketon gelöst. In diese Lösung werden 100 g Aluminium-Pigment (10%ige Dispersion in Ethylacetat) eingerührt. Das Beschichtungsmaterial wird mittels einer Rasterwalze (70 LPI x 127 µm) auf die Folie mit 2,7 Gew.% Pigment, bezogen auf die Folie, aufgetragen und anschließend bei 120 °C getrocknet. Es ergab sich eine Beschichtung, auf denen die Aluminium-Pigmente in Form punktueller Bereiche deutlich sichtbar waren.

### Vergleichsbeispiel 1

Zum Vergleich wurde ein Vergleichs-Glitter aus derselben Cellulose-haltigen Folie, die im Vakuumverfahren mit Aluminium (0,5 Gew.% im Vakuum) beschichtet und durch entsprechende Verfahren mit der gleichen Größe wie in Beispiel 1 hergestellt wurde, verwendet. Im Anschluss an die Vakuum-Beschichtung wurde eine Celluloseacetat-Beschichtung wie in Beispiel 1, allerdings ohne Aluminium-Pigment, aufgetragen. Hierbei ergab sich eine gleichmäßige Beschichtung.

### Testbeispiel 1: Glitterbeständigkeit

Mit den beiden Glittern (erfindungsgemäßer Glitter gemäß Beispiel 1 und Vergleichsglitter gemäß Vergleichsbeispiel 1) wurde die Beständigkeit gegenüber Wasser durch Einbringen in vollentsalztes Wasser (VE-Wasser) für 24 h bei 23°C getestet. In einem Probefläschchen wird der jeweilige Glitter vorgelegt und mit VE-Wasser überschichtet. Die Beurteilung erfolgt visuell. Die Ergebnisse sind in Tabelle 1 gezeigt.

**Tabelle 1: Wasserbeständigkeit der Glitter von Beispiel 1 und Vergleichsbeispiel 1**

| | **Erfindungsgemäßer Glitter (Beispiel 1)** | **Cellulose Glitter** mit **Vakuum-Metallisierung** |
|---|---|---|
| | | **SiLiglam PURE BIO SPARKLE 1610-50-2hex, Fa. Sigmund Lindner GmbH (Vergleichsbeispiel 1)** |
| VE-Wasser (24 h bei 23°C) | Keine sichtbare Veränderung | Glanzverlust |

Mit den beiden Materialien wurde zudem die Beständigkeit gegenüber 25%iger Ammoniak-Lösung in Wasser getestet. In einem Probefläschchen wird der jeweilige Glitter vorgelegt und mit Ammoniak-Lösung überschichtet. Die Beurteilung erfolgt visuell. Die Ergebnisse sind in Tabelle 2 gezeigt.

**Tabelle 2: Ammoniak-Beständigkeit der Glitter von Beispiel 1 und Vergleichsbeispiel 1**

| | **Erfindungsgemäßer Glitter (Beispiel 1)** | **Cellulose Glitter mit Vakuum-Metallisierung** |
|---|---|---|
| | | **SiLiglam PURE BIO SPARKLE 1610-50-2hex, Fa. Sigmund Lindner GmbH (Vergleichsbeispiel 1)** |
| 25%ige Ammoniak-Lösung | Nach 30 Minuten keine visuelle Veränderung | Glitter wird nach 5 Minuten transparent |

Beispiel 2a: Beschichtung im Wirbelschichtverfahren auf Cellulose-Partikel Transparente Glitterpartikel (SiLiglam PURE BIO SPARKLE 1610-11-2hex, Partikelgröße 0,2 mm, Fa. Sigmund Lindner GmbH), die aus einer Cellulose-Folie mit einer Dicke von 17 µm bis 23 µm hergestellt wurden, werden mittels Wirbelschichtverfahren mit Aluminium-Pigmenten beschichtet. Für die Herstellung des Beschichtungsmaterials werden 1000 g Schellack-Lösung (50%ig in Ethanol) vorgelegt. In diese Lösung werden 100 g Aluminium-Pigment-Dispersion (10%ige Dispersion in Ethylacetat) eingerührt.

3 kg transparente Glitterpartikel werden im Materialbehälter einer Wirbelschichtanlage (ProCell Lab, Fa. Glatt) vorgelegt und mittels einer auf 80°C vorgewärmter Prozessluft mit einem Luftdurchsatz von 35 m³/h verwirbelt, und es bildet sich ein Wirbelbett. Im sogenannten Bottom-Spray-Verfahren wird die Beschichtungslösung mit einer Sprührate von 10 ml/min in das Wirbelbett eingesprüht. Die so beschichteten Glitterpartikel werden aus der Maschine entnommen und auf Raumtemperatur abgekühlt. Die so hergestellten Glitterpartikel entsprechen dem in Fig. 4 dargestellten schematischen Aufbau.

Beispiel 2b: Beschichtung im Wirbelschichtverfahren auf Celluloseacetat-Partikel Analog zu Beispiel 2 a werden Glitter auf Basis des Cellulose-Derivats Celluloseacetat hergestellt. Transparente Glitterpartikel (SiLiglam PURE NATURE 1710-11-2hex, Partikelgröße 0,2 mm, Fa. Sigmund Lindner GmbH), die aus einer Celluloseacetat-Folie mit einer Dicke von 10 µm bis 15 µm hergestellt wurden, werden mittels Wirbelschichtverfahren mit Aluminium-Pigmenten beschichtet. Für die Herstellung des Beschichtungsmaterials werden 1000 g Schellack-Lösung (50%ig in Ethanol) vorgelegt. In diese Lösung werden 100 g Aluminium-Pigment-Dispersion (10%ige Dispersion in Ethylacetat) eingerührt.

3 kg transparente Glitterpartikel werden im Materialbehälter einer Wirbelschichtanlage (ProCell Lab, Fa. Glatt) vorgelegt und mittels einer auf 80°C vorgewärmter Prozessluft mit einem Luftdurchsatz von 35 m³/h verwirbelt, und es bildet sich ein Wirbelbett. Im sogenannten Bottom-Spray-Verfahren wird die Beschichtungslösung mit einer Sprührate von 10 ml/min in das Wirbelbett eingesprüht. Die so beschichteten Glitterpartikel werden aus der Maschine entnommen und auf Raumtemperatur abgekühlt. Die so hergestellten Glitterpartikel entsprechen dem in Fig. 4 dargestellten schematischen Aufbau.

### Vergleichsbeispiel 2

Zum Vergleich wurde Glitter aus Cellulose-haltiger Folie aus Beispiel 1, die im Vakuumverfahren mit Aluminium beschichtet und durch entsprechende Verfahren mit der gleichen Größe hergestellt wurde, verwendet. Nach dem Aufbringen des Aluminiums wurde eine Schellack-Lösung wie in Beispiel 2, jedoch ohne Aluminium, aufgebracht. Weiterhin wurde ein Glitter auf Basis der Celluloseacetat-Folie aus Beispiel 2b entsprechend mit Aluminium beschichtet und mit gleicher Größe hergestellt und eine Schellack-Lösung aufgebracht. Ebenso wurde als Referenz SiLiglam PURE BIO SPARKLE 1610-11-2hex aus Beispiel 2, welcher transparent ist, dem Beständigkeitstest unterzogen.

### Testbeispiel 2: Glitterbeständigkeit

Mit den Materialien aus Beispiel 2a, Beispiel 2b und Vergleichsbeispiel 2 wurde die Beständigkeit gegenüber wasserbasiertem Haargel (Balea MEN, Styling Gel maximum power, Fa. dm) getestet. In je einem Probefläschchen wird der Glitter vorgelegt und mit Haargel überschichtet. Die Proben werden bei 50 °C in einem Laborofen gelagert. Die Beurteilung erfolgt visuell. Nach einer Ofenlagerung von 4 Wochen ist keine sichtbare Veränderung des erfindungsgemäßen Glitters aus Beispiel 2a und des erfindungsgemäßen Glitters aus Beispiel 2b vorhanden. Beim Vergleichsglitter aus Vergleichsbeispiel 2 (sowohl mit Cellulose- als auch mit Celluloseacetat-Folie) hat eine vollständige Entfärbung stattgefunden, die darauf zurückzuführen ist, dass die im Vakuumverfahren aufgebrachte Aluminiumschicht in Lösung gegangen ist. Beim Glitter SiLiglam PURE BIO SPARKLE 1610-11-2hex ergab sich keine visuelle Veränderung und der Glitter war von Anfang bis Ende transparent.

### Beispiel 3: Beschichtung im Wirbelschichtverfahren

Transparente Glitterpartikel (SiLiglam PURE BIO SPARKLE 1604-11-2hex, Partikelgröße 1,0 mm, Fa. Sigmund Lindner GmbH), die aus einer Cellulose-Folie mit einer Dicke von 17 µm bis 23 µm hergestellt wurden, werden mittels Wirbelschichtverfahren mit Aluminium-Pigmenten beschichtet. Für die Herstellung des Beschichtungsmaterials werden 250 g Polyurethan-Dispersion vorgelegt. In diese Dispersion werden 1150 g Aluminium-Pigment-Dispersion (10%ige Dispersion in Isopropylalkohol) eingerührt.

3 kg transparente Glitterpartikel werden im Materialbehälter einer Wirbelschichtanlage (ProCell Lab, Fa. Glatt) vorgelegt und mittels einer auf 80°C vorgewärmter Prozessluft mit einem Luftdurchsatz von 35 m³/h verwirbelt, und es bildet sich ein Wirbelbett. Im sogenannten Bottom-Spray-Verfahren wird die Beschichtungslösung mit einer Sprührate von 10 ml/min in das Wirbelbett eingesprüht. Die so beschichteten Glitterpartikel werden aus der Maschine entnommen und auf Raumtemperatur abgekühlt.

### Vergleichsbeispiel 3

Zum Vergleich wurde ein kommerziell erhältlicher Glitter (Fa. Ronald Britton, BioGlitter Sparcle, silver, 0.040) auf Basis von regenerierter Cellulose in der gleichen Partikelgröße wie der erfindungsgemäße Glitter verwendet.

### Testbeispiel 3: Glitterbeständigkeit

Mit den beiden Materialien aus Beispiel 3 und Vergleichsbeispiel 3 wurde die Formbeständigkeit in Wasser durch Einbringen in vollentsalztes Wasser (VE-Wasser) für 30 Minuten bei 50°C getestet. In je einem Probefläschchen wird der Glitter vorgelegt und mit VE-Wasser überschichtet. Die Beurteilung erfolgt visuell. Die Ergebnisse sind in Tabelle 3 gezeigt.

**Tabelle 3: Formbeständigkeit der Glitter von Beispiel 3 und Vergleichsbeispiel 3**

| | **Erfindungsgemäßer Glitter (Beispiel 3)** | **Cellulose Glitter mit Vakuum-Metallisierung** |
|---|---|---|
| | | **BioGlitter Sparkle, silver, 0.040, Fa. Ronald Britton (Vergleichsbeispiel 3)** |
| VE-Wasser (30 min bei 50°C) | Keine sichtbare Veränderung | Sichtbare Verformung und Einrollen der Glitterpartikel. |

### Beispiel 4: Applikationsbeispiele erfindungsgemäßer Glitter

### Produkt 1: Haarwachs

| **Phase** | **Inhaltsstoff** | **%W/W** |
|---|---|---|
| **A** | Wasser | 45,5 |
| | Propylenglykol | 4,0 |
| | PVP (Polyvinylpyrrolidon) | 6,0 |
| | Erfindungsgemäßer Glitter gemäß Beispiel 2 | 2,0 |
| **B** | Cera Alba, Ceteareth-25 | 38 |
| | Octyldodecanol | 4 |
| | Phenoxyethanol (und) Ethylhexylglycerin | 0,5 |
| | **Summe** | 100,0 |

### Herstellung:

Phasen A und B jeweils getrennt mischen und so lange rühren, bis eine homogene Lösung entsteht. Phasen A und B getrennt voneinander auf 90 - 95 °C erhitzen. Beiden Phasen unter Rühren mischen und abkühlen lassen.

Lagerstabilität: > 4 Wochen bei 50 °C.

### Produkt 2: Bastelklebstoff

| **Inhaltsstoff** | **%W/W** |
|---|---|
| Papierleim ÖkoNorm Pro Coll (Glucose-Saccharid-Gemisch, Saccarose, Wasser, Glycerin, Phenoxyethanol) | 98,5 |
| Erfindungsgemäßer Glitter gemäß Beispiel 2 | 1,5 |
| **Summe** | 100,0 |

### Herstellung:

Glitter unter Rühren zu dem Klebstoff dazugeben und homogenisieren.

Lagerstabilität: > 4 Wochen bei 50 °C.

### Produkt 3: Glanzlack, wasserverdünnbar

| **Inhaltsstoff** | **%W/W** |
|---|---|
| Alberdingk AC 2742, Fa. Alberdingk-Boley | 71,6 |
| 2-Butoxyethanol | 5 |
| Wasser | 19 |
| Byk 333, Fa. Byk | 3 |
| Byk 349, Fa. Byk | 0,2 |
| DSX 1514, Fa. BASF | 0,2 |
| Erfindungsgemäßer Glitter gemäß Beispiel 2 | 1,0 |
| **Summe** | 100,0 |

### Herstellung:

Inhaltsstoffe unter Rühren mischen

### Lagerstabilität: > 4 Wochen bei 50 °C.

### Produkt 4: Spielknete

| **Inhaltsstoff** | **%W/W** |
|---|---|
| Kinder Soft Knete gelb, Fa. Feuchtmann | 99 |
| Erfindungsgemäßer Glitter gemäß Beispiel 2 | 1 |
| **Summe** | 100,0 |

### Herstellung:

Glitter zu der Spielknete dazugeben und homogenisieren.

Lagerstabilität: > 4 Wochen bei 50 °C.

## Patentansprüche

1. Beschichteter Glitter, umfassend eine Folie, die eine Beschichtung umfassend Metall-Pigmente, insbesondere Aluminium-Pigmente, aufweist, wobei die Folie Cellulose und/oder ein Derivat davon umfasst.

2. Beschichteter Glitter nach Anspruch 1, wobei die Folie wenigstens 60 Gew.% Cellulose und/oder ein Derivat davon umfasst, bezogen auf das Gewicht der Folie.

3. Beschichteter Glitter nach Anspruch 1 oder 2, wobei die Folie im Wesentlichen aus Cellulose und/oder einem Derivat davon besteht.

4. Beschichteter Glitter nach einem der Ansprüche 1 bis 3, wobei die Beschichtung eine Trockenschichtdicke von 0,1 µm bis 10 µm aufweist.

5. Beschichteter Glitter nach einem der Ansprüche 1 bis 4, wobei der Glitter 0,01 - 10 Gew.% Metall-Pigmente, insbesondere Aluminium-Pigmente, aufweist, bezogen auf das Gewicht der Folie.

6. Verwendung von beschichteten Glitter gemäß einem der Ansprüche 1 - 5 in einem kosmetischen Produkt, in einem Beschichtungswerkstoff, in einem Klebstoff, oder in einer Knet- und/oder Formmasse.

7. Kosmetisches Produkt, umfassend einen beschichteten Glitter gemäß einem der Ansprüche 1 - 5.

8. Beschichtungswerkstoff, umfassend einen beschichteten Glitter gemäß einem der Ansprüche 1 - 5.

9. Klebstoff, umfassend einen beschichteten Glitter gemäß einem der Ansprüche 1 -5.

10. Knet- und/oder Formmasse, umfassend einen beschichteten Glitter gemäß einem der Ansprüche 1 - 5.

11. Verfahren zur Herstellung eines beschichteten Glitters umfassend:
- Bereitstellen einer Folie, wobei die Folie Cellulose und/oder ein Derivat davon umfasst;
- Herstellen einer Beschichtung umfassend Metall-Pigmente, insbesondere Aluminium-Pigmente, auf mindestens einer Seite der Folie; und
- Herstellung des beschichteten Glitters; oder
- Bereitstellen einer Folie, wobei die Folie Cellulose und/oder ein Derivat davon umfasst;
- Schneiden der Folie zu unbeschichteten Glitter-Partikeln geeigneter Größe; und
- Beschichtung der unbeschichteten Glitter-Partikel mit Metall-Pigmenten, insbesondere Aluminium-Pigmenten.

12. Verfahren nach Anspruch 11, wobei die Beschichtung mit Metall-Pigmenten, insbesondere Aluminium-Pigmenten, aus flüssiger Phase, insbesondere auf der Folie, erfolgt.
